# EUROPEAN PATENT SPECIFICATION

(11) **EP 3 808 323 B1**
(45) Date of publication and mention of the grant of the patent: **30.03.2022**
(21) Application number: 20201913.9
(22) Date of filing: 15.10.2020
(51) Int. Cl.: A61H 1/02, A61F 5/042

(54) **STRETCHER ARRANGEMENT FOR APPLYING A STRETCHING MOVEMENT TO A PERSON**
TRAGENANORDNUNG ZUR ANWENDUNG EINER STRECKBEWEGUNG AN EINER PERSON
AGENCEMENT DE CIVIÈRE POUR APPLIQUER UN MOUVEMENT D'ÉTIREMENT À UNE PERSONNE

(30) Priority: 15.10.2019 SE 1951165
(43) Date of publication of application: 21.04.2021
(73) Proprietor: LLD Nybohov Invest AB, 117 63 Stockholm (SE)
(72) Inventor: LUNDBLAD, Leif J.I., 115 21 Stockholm (SE)
(74) Representative: Bjerkéns Patentbyrå KB (Stockholm)

(56) References cited:
- WO-A1-00/09065
- WO-A1-2013/087001
- WO-A1-2016/183458
- CN-U- 201 755 264
- KR-A- 20150 043 674
- US-A- 2 693 796

## Description

### Technical field

The present disclosure relates to a stretcher arrangement, specifically adapted to be arranged on a bed for improving health and well-being and also for treatment of spine and/or joint problems, and also for preventing such problems.

### Background

The discs between the vertebras in the spine need to have a rest to recover every day. The optimal recovery time is around 12 hours, but most people sleep only 6-8 hours, which thus can lead to back problems due to too short rest for the back. The discs may then be too thin since they have not absorbed enough liquid needed for an active day.

Below is listed some patent documents disclosing various technologies within this technical field.

DE10246760 describes a bed comprising a fixed part for the head and upper torso and a movable part for the bottom and legs. Further, the bed comprises rollers, which pull the movable part away from the fixed part meanwhile the person is sleeping.

US20120265114 shows a stretcher, which is arranged on one side of the upper surface of a bed. The stretcher may stretch the lower body of a person and the stretching may be performed for a whole night during sleep.

JP10023947 shows a health bed comprising a vertebrae stretching mattress. The aim is to be treated while sleeping. The device in this document seems to use pads that are fixed on predetermined places in the mattresses, e.g. under the back.

JP2001333947 shows a machine to stretching out a person's back comprising a sleeping bed which is formed by two parts. The two parts are pivotally connected to each other.

KR 2015 0043674 relates to a vertebra pulling apparatus that comprises drawing means to towing upper and lower side connection members connected and fixed to pelvis belt attached to the pelvis region of a patient lying on a bed.

An object of the present invention is to achieve an easy to use arrangement that provides improved therapeutic and recovery effects, and also improved health and well-being, in comparison to the presently known devices.

### Summary

The above-mentioned objects are achieved by the present invention according to the independent claim.

Preferred embodiments are set forth in the dependent claims.

The present invention relates to aspects of a stretcher arrangement (2) comprising a first sheet (4) and a second sheet (6) to be arranged on a bed (8) having a first end (10) and a second end (12), and a length L along a longitudinal axis A.

The first sheet (4) comprises a first surface (14), being a part of the first sheet that faces upward when the sheet is arranged on the bed (8).

The second sheet (6) comprises a second surface (16), being a part of the second sheet (6) that faces upward when the sheet is arranged on the bed (8), wherein said first surface (14) and said second surface (16) cover different parts of the bed surface, and border each other along a border line B that at least partly is essentially perpendicular to said axis A.

The stretcher arrangement (2) further comprises:
- a sheet movement unit (18) structured to apply movement to at least one of said first and second sheets (4, 6) essentially along the longitudinal axis A of said bed;
- a control unit (20) configured to control said sheet movement unit (18), according to a set of sheet movement rules, such that a relative movement between said first surface (14) and said second surface (16) is provided, moving these surfaces apart from each other, resulting in that a stretching movement is applied to a person lying on the sheets.

Thus, the stretcher arrangement comprises two sheets placed on a bed. One sheet is placed on the upper part of the bed and the other on the lower part of the bed. The sheet arranged on the lower part of the bed is connected to a sheet movement unit comprising a roller. When the person has fallen asleep, and/or the procedure is initiated, the lower sheet will be slowly rolled up at a predetermined variable rolling speed and thus, the spine of the person sleeping/lying in bed will be stretched.

A relative movement is thereby provided between the first and second sheets arranged on a bed such that stretching is applied to the person lying on the bed. One important advantage of the stretcher arrangement according to the present invention is that the person lying on the bed does not have to be attached to any attachment means, but only lie on the bed.

### Brief description of the drawings

Figure 1 is a schematic illustration of a side view of a stretcher arrangement according to an example not being part of the present invention arranged on a bed.
Figure 2 is a schematic illustration of a view from above of a stretcher arrangement according to the present invention arranged on a bed.
Figure 3 is a schematic illustration of a side view of a stretcher arrangement according to one embodiment of the present invention arranged on a bed.
Figure 4 is a schematic illustration of a view from above of a stretcher arrangement according to one embodiment of the present invention arranged on a bed.

### Detailed description

The stretcher arrangement will now be described in detail with references to the appended figures. Throughout the figures the same, or similar, items have the same reference signs. Moreover, the items and the figures are not necessarily to scale, emphasis instead being placed upon illustrating the principles of the invention.

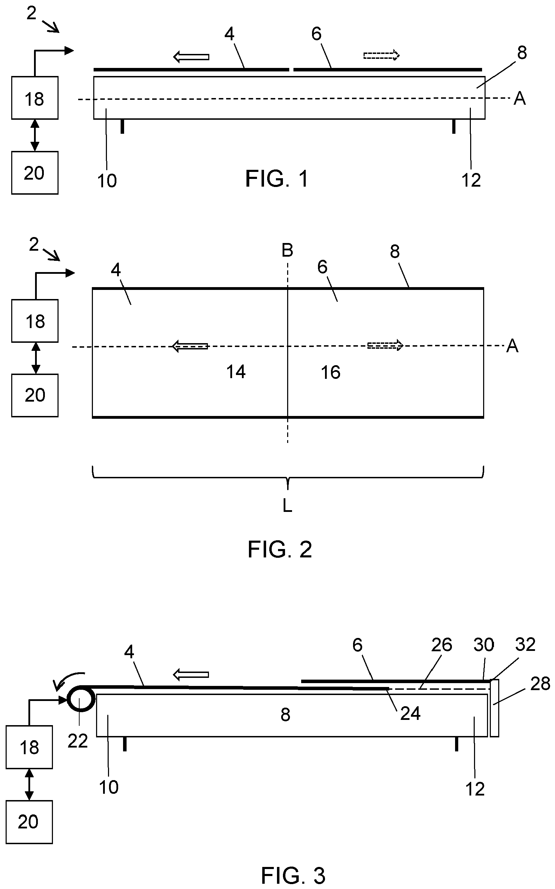

First with reference to the schematic illustrations of figures 1 and 2, a stretcher arrangement 2 is provided, comprising a first sheet 4 and a second sheet 6 to be arranged on a bed 8 having a first end 10 and a second end 12, and a length L (e.g. 200 or 210 cm) along a longitudinal axis A.

The first sheet 4 comprises a first surface 14, being a part of the first sheet that faces upward when the sheet is arranged on the bed 8, and the second sheet 6 comprises a second surface 16, being a part of the second sheet 6 that faces upward when the sheet is arranged on the bed 8.

The first surface 14 and the second surface 16 cover different parts of the bed surface along a border line B that at least partly is essentially perpendicular to said axis A.

The stretcher arrangement 2 further comprises a sheet movement unit 18 structured to apply movement to at least one of the first and second sheets 4, 6 essentially along the longitudinal axis A of the bed. The stretcher arrangement also comprises a control unit 20 configured to control the sheet movement unit 18, according to a set of sheet movement rules, such that a relative movement between the first surface 14 and the second surface 16 is provided, moving these surfaces apart from each other, resulting in that a stretching movement is applied to a person lying on the sheets. The sheet movement rules comprise at least one of the following rules:
Applying a stretching movement rate: e.g. 1-10 cm per hour, may be variable.
Applying a movement program: periodical movement, e.g. movement e.g. every 0.5 hours. Starting of program: delay e.g. 20, 40 or 60 minutes.

As one example, one of the first and second sheets is moved and the other is kept still. In figures 1 and 2 a solid arrow is used to indicate that the first sheet 4 is intended to be moved and the second sheet 6 is kept still. The dashed arrow on the second sheet 6 indicates that also this sheet 6 may be moved, at the same time as the first sheet is moved. Preferably, the first surface 14 and said second surface 16 together cover essentially the entire bed surface.

The border line B is advantageously provided at a position in the range of 0.4 - 0.6 L. In the figure the border line B is shown approximately at a middle position along axis A, i.e. at 0.5 L. The position of the border line B may be such that a person lying on the bed has his/her waistline approximately on the border line B.

According to one embodiment, which is illustrated in figure 3, the sheet movement unit 18 is structured to be arranged in connection with at least one of the first and second ends 10, 12 of the bed, and comprises at least one roller 22 to be arranged essentially perpendicular to the longitudinal axis A. The control unit 20 is configured to control the rotation of the at least one roller 22, such that at least one of the first and second sheets 4, 6 is slowly wound up on the at least one roller 22, resulting in that the first and second surfaces slowly move in relation to each other.

The roller 22 is driven by an electrical motor having a capability of generating the necessary force to apply the stretching movement to the sheet. The rotational force needed to be applied by the motor is dependent upon the weight of the person lying on the sheets. The motor is thereby configured to generate a variable output torque in dependence of the weight. The weight may be manually entered to the control unit, or may be indirectly determined by measuring the current consumed by the motor.

In the figures only one sheet movement unit is shown. Naturally, another sheet movement unit, including a roller may be arranged at the opposite end of the bed being adapted to apply movement to the other sheet.

Furthermore, herein the sheet movement unit is exemplified to comprise a motor and a roller. Naturally, other means to provide movement to the sheet(s) may be considered, e.g. an elastic force generating arrangement provided to generate movement force to the sheet(s).

According to the invention, the first sheet 4 is configured to be arranged on the bed 8 and to extend from one of the first and second ends 10, 12 of the bed 8, and the second sheet 6 has a length that covers a predetermined part of the bed 8. The predetermined part may be in the range of 30-70%, and more preferred 40-60%, e.g. 50%, of the entire bed area. The second sheet 6 is configured to be arranged on the first sheet 4 such that it covers, i.e. overlaps, at least a part of the first sheet 4, and to extend from the other of said first and second ends 10, 12 of the bed.

The sheet movement unit 18 is structured to apply a movement to the first sheet 4 such that it is moved in a direction away from the other of said first and second ends 10, 12 of the bed.

In a further embodiment, illustrated in figure 3, a first short side 24 of the first sheet 4 is provided with an elastic attachment member 26 structured to be attached to an attachment arrangement 28 configured to be arranged in connection the other of the first and second ends 10, 12 of the bed. Thereby the first sheet 4 is remained stretched during the slow movement applied by the sheet movement unit 18, and also, when the movement procedure has been finalized and the person has left the bed, the first sheet 4 may return to its initial position by forces applied from the elastic attachment member 26, i.e. move to the right in figure 3. The elastic attachment member 26 may comprise a number of flat, flexible and elastic bands, e.g. rubber bands.

Preferably, the second sheet 6 has a first short side 30 provided with a second sheet attachment member 32 structured to be attached to the attachment arrangement 28 such that the second sheet 6 is arranged on top of the first sheet 4, and remains in its position during movement of the first sheet 4 (to the left figure 3).

According to another embodiment, at least one of the first and second sheets 4, 6 is provided with a low frictional material on the side(s) facing downward when the sheets are arranged at the bed. This low frictional material may e.g. be a Teflon-like material, or a silk material. This feature will further facilitates easy movement of the sheet as the friction to the underlying upper surface of the bed is reduced.

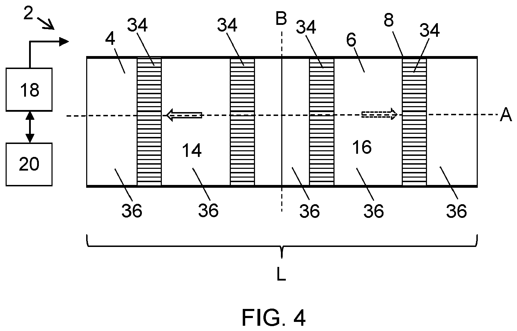

According to still another embodiment, and with references to figure 4, at least one of the first and second sheets 4, 6 is provided with one or many elastic sections 34, and wherein an elastic section has a higher elasticity along axis A in comparison to non-elastic sections 36 of the at least one of said first and second sheets 4, 6. It is then possible to tailor the sheet(s) to a person's specific needs. For example, it may then be advantageous to position the sheet such that one elastic section essentially is positioned where the person's knees are lying if the person has knee problems. During movement of a sheet the non-elastic sections 36 will move apart from each other.

The control unit 20 is configured to control the sheet movement unit 18 and is configured to receive user input via a user input interface. The user input interface may be integrated into the control unit or attached to the control unit via a wire. As an alternative a remote input interface may be provided, e.g. as a remote control unit, configured to communicate with the control unit via a wireless interface, e.g. via an infrared (IR) communication protocol, via Bluetooth, or via an internet protocol. The remote input interface may also be an application program downloaded on a smartphone. The user input interface facilitates the user to easily choose any available movement rule and set a plurality of different parameters in order to tailor the movement to his/her specific needs.

## Claims

1. A stretcher arrangement (2) comprising a first sheet (4) and a second sheet (6) to be arranged on a bed (8) having a first end (10) and a second end (12), and a length L along a longitudinal axis A, wherein the lenght corresponding to the lenght of the bed;
the first sheet (4) comprises a first surface (14), being a part of the first sheet that faces upward when the first sheet is arranged on the bed (8),
the second sheet (6) comprises a second surface (16), being a part of the second sheet (6) that faces upward when the second sheet is arranged on the bed (8),
wherein said first surface (14) and said second surface (16) cover different parts of the bed surface along a border line B that at least partly is perpendicular to said axis A, and that said stretcher arrangement (2) further comprises:
- a sheet movement unit (18) structured to apply movement to at least one of said first and second sheets (4, 6) along the longitudinal axis A of said bed;
- a control unit (20) configured to control said sheet movement unit (18), according to a set of sheet movement rules, wherein a relative movement between said first surface (14) and said second surface (16) is provided, moving these surfaces apart from each other, wherein said relative movement is provided to apply a stretching movement to a person lying on the sheets, **characterized in that** the first sheet (4) is configured to be arranged on said bed (8) and to extend from one of said first and second ends (10, 12) of the bed (8), and the second sheet (6) has a length that covers a predetermined part of said bed (8), and that said second sheet (6) is configured to be arranged on said first sheet (4) such that said second sheet (6) covers at least a part of said first sheet (4), and to extend from the other of said first and second ends (10, 12) of the bed.

2. The stretcher arrangement (2) according to claim 1, wherein said first surface (14) and said second surface (16) together cover the entire bed surface.

3. The stretcher arrangement (2) according to claim 1 or 2, wherein said border line B is provided at a position in the range of 0.3 - 0.7 L, preferably 0.4 - 0.6 L, wherein L is the length of the bed.

4. The stretcher arrangement (2) according to any of claims 1-3, wherein said sheet movement unit (18) is structured to be arranged in connection with at least one of said first and second ends (10, 12) of said bed, and comprises at least one roller (22) to be arranged perpendicular to said longitudinal axis A, wherein said control unit (20) is configured to control the rotation of said at least one roller (22) to slowly wind up said at least one of said first and second sheets (4, 6) on said at least one roller (22), wherein said first and second surfaces (14, 16) slowly move in relation to each other.

5. The stretcher arrangement (2) according to any of claims 1-4, wherein said first sheet (4) is moved in a direction away from the other of said first and second ends (10, 12) of the bed.

6. The stretcher arrangement (2) according to any of claims 1-5, wherein a first short side (24) of said first sheet (4) is provided with an elastic attachment member (26) structured to be attached to an attachment arrangement (28) configured to be arranged in connection the other of said first and second ends (10, 12) of said bed.

7. The stretcher arrangement (2) according to claim 6, wherein the second sheet (6) has a first short side (30) provided with a second sheet attachment member (32) structured to be attached to said attachment arrangement (28) such that said second sheet (6) is arranged on top of said first sheet (4).

8. The stretcher arrangement (2) according to any of claims 1-7, wherein at least one of said first and second sheets (4, 6) is provided with one or many elastic sections (34), and wherein an elastic section has a higher elasticity along axis A in comparison to non-elastic sections (36) of said at least one of said first and second sheets (4, 6).

## Patentansprüche

1. Tragenanordnung (2), umfassend ein erstes Laken (4) und ein zweites Laken (6), die auf einem Bett (8) mit einem ersten Ende (10) und einem zweiten Ende (12) anzuordnen sind, und eine Länge L entlang einer Längsachse A, wobei die Länge der Länge des Betts entspricht;
wobei das erste Laken (4) eine erste Oberfläche (14) umfasst, die ein Teil des ersten Lakens ist und nach oben zeigt, wenn das erste Laken auf dem Bett (8) angeordnet ist,
wobei das zweite Laken (6) eine zweite Oberfläche (16) umfasst, die ein Teil des zweiten Lakens (6) ist und nach oben zeigt, wenn das zweite Laken auf dem Bett (8) angeordnet ist,
wobei die erste Oberfläche (14) und die zweite Oberfläche (16) unterschiedliche Teile der Bettoberfläche entlang einer Grenzlinie B bedecken, die mindestens teilweise senkrecht zu der Achse A ist, und dass die Tragenanordnung (2) ferner Folgendes umfasst:
- eine Lakenbewegungseinheit (18), die dazu strukturiert ist, eine Bewegung auf mindestens eines des ersten und des zweiten Lakens (4, 6) entlang der Längsachse A des Betts anzuwenden;
- eine Steuereinheit (20), die dazu konfiguriert ist, die Lakenbewegungseinheit (18) gemäß einem Satz von Lakenbewegungsregeln zu steuern, wobei eine relative Bewegung zwischen der ersten Oberfläche (14) und der zweiten Oberfläche (16) bereitgestellt ist, durch welche die Oberflächen voneinander wegbewegt werden, wobei die relative Bewegung dazu bereitgestellt ist, eine Streckbewegung auf eine auf den Laken liegende Person anzuwenden, **dadurch gekennzeichnet, dass** das erste Laken (4) dazu konfiguriert ist, auf dem Bett (8) angeordnet zu werden und sich von einem des ersten und des zweiten Endes (10, 12) des Betts (8) zu erstrecken, und das zweite Laken (6) eine Länge aufweist, die einen vorbestimmten Teil des Bettes (8) bedeckt, und dadurch, dass das zweite Laken (6) dazu konfiguriert ist, auf dem ersten Laken (4) angeordnet zu werden, sodass das zweite Laken (6) mindestens einen Teil des ersten Lakens (4) bedeckt, und sich von dem anderen des ersten und des zweiten Endes (10, 12) des Betts zu erstrecken.

2. Tragenanordnung (2) nach Anspruch 1, wobei die erste Oberfläche (14) und die zweite Oberfläche (16) gemeinsam die gesamte Bettoberfläche bedecken.

3. Tragenanordnung (2) nach Anspruch 1 oder 2, wobei die Grenzlinie B an einer Position im Bereich von 0,3-0,7 L, vorzugsweise 0,4-0,6 L, bereitgestellt ist, wobei L die Länge des Betts ist.

4. Tragenanordnung (2) nach einem der Ansprüche 1-3, wobei die Lakenbewegungseinheit (18) dazu strukturiert ist, in Verbindung mit mindestens einem des ersten und des zweiten Endes (10, 12) des Betts angeordnet zu werden, und mindestens eine Rolle (22) umfasst, die senkrecht zu der Längsachse A anzuordnen ist, wobei die Steuereinheit (20) dazu konfiguriert ist, die Rotation der mindestens einen Rolle (22) so zu steuern, dass das mindestens eine des ersten und des zweiten Lakens (4, 6) auf der mindestens einen Rolle (22) langsam aufgewickelt wird, wobei sich die erste und die zweite Oberfläche (14, 16) langsam in Bezug aufeinander bewegen.

5. Tragenanordnung (2) nach einem der Ansprüche 1-4, wobei das erste Laken (4) in eine Richtung weg von dem anderen des ersten und des zweiten Endes (10, 12) des Betts bewegt wird.

6. Tragenanordnung (2) nach einem der Ansprüche 1-5, wobei eine erste kurze Seite (24) des ersten Lakens (4) mit einem elastischen Anbringungselement (26) bereitgestellt ist, das dazu strukturiert ist, an einer Anbringungsanordnung (28) angebracht zu werden, die dazu konfiguriert ist, in Verbindung mit dem anderen des ersten und des zweiten Endes (10, 12) des Betts angeordnet zu werden.

7. Tragenanordnung (2) nach Anspruch 6, wobei das zweite Laken (6) eine erste kurze Seite (30) aufweist, die mit einem Anbringungselement (32) des zweiten Lakens bereitgestellt ist, das dazu strukturiert ist, an der Anbringungsanordnung (28) angebracht zu werden, sodass das zweite Laken (6) auf dem ersten Laken (4) angeordnet ist.

8. Tragenanordnung (2) nach einem der Ansprüche 1-7, wobei mindestens eines des ersten und des zweiten Lakens (4, 6) mit einem oder vielen elastischen Abschnitten (34) bereitgestellt ist und wobei ein elastischer Abschnitt eine höhere Elastizität entlang der Achse A im Vergleich zu nicht elastischen Abschnitten (36) des mindestens einen des ersten und des zweiten Lakens (4, 6) aufweist.

## Revendications

1. Agencement de brancard (2) comprenant une première feuille (4) et une seconde feuille (6) à agencer sur un lit (8) ayant une première extrémité (10) et une seconde extrémité (12), et une longueur L le long d'un axe longitudinal A, la longueur correspondant à la longueur du lit ;
la première feuille (4) comprend une première surface (14), étant une partie de la première feuille qui est tournée vers le haut lorsque la première feuille est agencée sur le lit (8),
la seconde feuille (6) comprend une seconde surface (16), étant une partie de la seconde feuille (6) qui est tournée vers le haut lorsque la seconde feuille est agencée sur le lit (8),
dans lequel ladite première surface (14) et ladite seconde surface (16) recouvrent différentes parties de la surface de lit le long d'une ligne de bordure B qui est au moins partiellement perpendiculaire audit axe A, et en ce que ledit agencement de brancard (2) comprend en outre :
- une unité de mouvement de feuille (18) structurée pour appliquer un mouvement à au moins une desdites première et seconde feuilles (4, 6) le long de l'axe longitudinal A dudit lit ;
- une unité de commande (20) configurée pour commander ladite unité de mouvement de feuille (18), selon un ensemble de règles de mouvement de feuille, dans lequel un mouvement relatif entre ladite première surface (14) et ladite seconde surface (16) est prévu, déplaçant ces surfaces à distance l'une de l'autre, dans lequel ledit mouvement relatif est prévu pour appliquer un mouvement d'étirement à une personne allongée sur les feuilles, **caractérisé en ce que** la première feuille (4) est configurée pour être agencée sur ledit lit (8) et pour s'étendre depuis l'une desdites première et seconde extrémités (10, 12) du lit (8), et la seconde feuille (6) a une longueur qui recouvre une partie prédéterminée dudit lit (8), et **en ce que** ladite seconde feuille (6) est configurée pour être agencée sur ladite première feuille (4) de telle sorte que ladite seconde feuille (6) recouvre au moins une partie de ladite première feuille (4), et pour s'étendre à partir de l'autre desdites première et seconde extrémités (10, 12) du lit.

2. Agencement de brancard (2) selon la revendication 1, dans lequel ladite première surface (14) et ladite seconde surface (16) recouvrent ensemble la totalité de la surface du lit.

3. Agencement de brancard (2) selon la revendication 1 ou 2, dans lequel ladite ligne de bordure B est prévue à une position dans la plage de 0,3 à 0,7 L, de préférence de 0,4 à 0,6 L, dans lequel L est la longueur du lit.

4. Agencement de brancard (2) selon l'une quelconque des revendications 1 à 3, dans lequel ladite unité de mouvement de feuille (18) est structurée pour être agencée en liaison avec au moins une desdites première et seconde extrémités (10, 12) dudit lit, et comprend au moins un rouleau (22) devant être agencé perpendiculairement audit axe longitudinal A, dans lequel ladite unité de commande (20) est configurée pour commander la rotation dudit au moins un rouleau (22) pour enrouler lentement ladite au moins une desdites première et seconde feuilles (4, 6) sur ledit au moins un rouleau (22), dans lequel lesdites première et seconde surfaces (14, 16) se déplacent lentement l'une par rapport à l'autre.

5. Agencement de brancard (2) selon l'une quelconque des revendications 1 à 4, dans lequel ladite première feuille (4) est déplacée dans une direction s'éloignant de l'autre desdites première et seconde extrémités (10, 12) du lit.

6. Agencement de brancard (2) selon l'une quelconque des revendications 1 à 5, dans lequel un premier côté court (24) de ladite première feuille (4) est pourvu d'un élément de fixation élastique (26) structuré pour être fixé à un agencement de fixation (28) configuré pour être agencé en connexion avec l'autre desdites première et seconde extrémités (10, 12) dudit lit.

7. Agencement de brancard (2) selon la revendication 6, dans lequel la seconde feuille (6) a un premier côté court (30) muni d'un second élément de fixation de feuille (32) structuré pour être fixé audit agencement de fixation (28) de telle sorte que ladite seconde feuille (6) est agencée sur le dessus de ladite première feuille (4).

8. Agencement de brancard (2) selon l'une quelconque des revendications 1 à 7, dans lequel au moins l'une desdites première et seconde feuilles (4, 6) est pourvue d'une ou de plusieurs sections élastiques (34), et dans lequel une section élastique a une élasticité plus élevée le long de l'axe A par comparaison à des sections non élastiques (36) de ladite au moins une desdites première et seconde feuilles (4, 6).
